# EUROPEAN PATENT APPLICATION

(11) **EP 3 822 341 A1**
(43) Date of publication of application: **19.05.2021**
(21) Application number: 19811432.4
(22) Date of filing: 13.05.2019
(51) Int. Cl.: C12N 5/0783

(54) **EXPANSION AND CULTURE OF HUMAN-DERIVED NATURAL KILLER CELLS BY USING IGFBP2**

(30) Priority: 31.05.2018 KR 20180062611
(71) Applicant: Korea University Research and Business Foundation, Seoul 02841 (KR)
(72) Inventor: LEE, Kyung-Mi, Seoul 06001 (KR); SHIN, Min Hwa, Seoul 02638 (KR)
(74) Representative: Mewburn Ellis LLP
(86) International application number: PCT/KR2019/005726
(87) International publication number: WO 2019/231139

(57) **Abstract**

The present invention relates to a method for the expansion and culture of natural killer cells. By using a method for the expansion and culture of natural killer cells, according to the present invention, the proliferation of human-derived natural killer cells is remarkably enhanced such that natural killer cells to be used for cancer treatment can be efficiently obtained.

## Description

### Technical Field

The present invention relates to a method for culturing of expansion of natural killer cells, and more particularly to a method for culturing of expansion of natural killer cells comprising culturing natural killer cells in a medium containing IGFBP2.

### Background Art

Various intractable diseases such as cancer, which are difficult to cure with modern medicine, are caused by imbalances in the immune system due to abnormalities in the complex human immune system. Therefore, in recent years, studies have been actively conducted on the development of immunotherapeutic agents to treat various diseases by resolving imbalances in the immune system and restoring the immune system to a normal state.

Among immune cells, T cells have antigen specificity, but natural killer cells have an important characteristic of being activated first when foreign substances penetrate from the outside. In addition, in order to use T cells as cell therapy products, only autologous T cells may be used. However, in order to use natural killer cells as cell therapy products, natural killer cells from other healthy people may be used. Thus, natural killer cells have research value as immune cell therapy products for cancer treatment and are also highly likely to be used. Since natural killer cells exist in small amounts in the body compared to other immune cells, a large number of natural killer cells should be obtained for use as immune cell therapy products.

Since the number of natural killer cells in most cancer patients is very small, the cancer cell killing ability thereof is also significantly low. In particular, natural killer cells do not properly proliferate and exhibit anticancer functions under hypoxic conditions, which are typical characteristics of tumor microenvironments.

Since most intractable solid cancers have hypoxic regions, a method of expanding natural killer cells under such hypoxic conditions is needed for effective treatment of solid cancer patients.

The present invention has been made by paying attention to a previous invention, which teaches that, when natural killer cells are cultured under hypoxic conditions, proliferation and anticancer activity thereof are low, but when cells that have actively started to proliferate under normal conditions are placed in hypoxic conditions, proliferation thereof is remarkably increased (see Korean Patent Publication No. 2017-0124467).

The present inventors have made efforts to develop a method of effectively enhancing expansion of natural killer cells in the resting state under hypoxic conditions, and as a result, have found that, when natural killer cells in the resting state are treated with insulin-like growth factor-binding protein 2 (IGFBP2) during *ex vivo* expansion culture thereof, expansion of the natural killer cells is significantly enhanced not only under normal conditions, but also under hypoxic conditions, thereby completing the present invention.

### Summary of the Invention

An object of the present invention is to provide a method for culturing of effectively enhancing expansion of natural killer cells.

To achieve the above object, the present invention provides a culture method for expansion of natural killer cells comprising culturing natural killer cells in a medium containing IGFBP2.

### Brief Description of Drawings

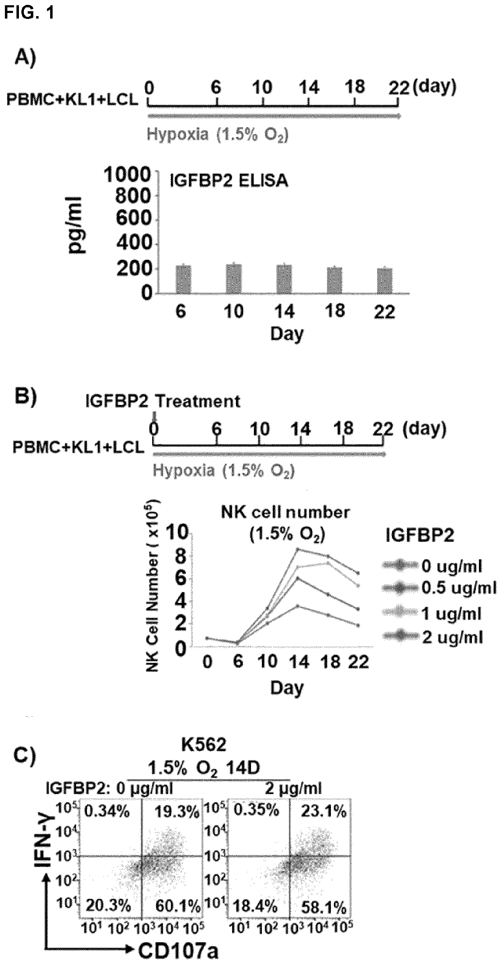

FIG. 1 shows the results of analyzing the effect of IGFBP2 on the growth of natural killer cells in the resting state under a hypoxic condition. FIG. 1(A) shows a method of expanding natural killer cells under a hypoxic condition, and shows the results of measuring secreted IGFBP2 by ELISA (Enzyme-Linked ImmunoSorbent Assay), FIG. 1(B) shows a method for IGFBP2 treatment and culture under a hypoxic condition, and shows a growth curve of natural killer cells, and FIG. 1(C) shows the results of analyzing the expression of CD107a (a measure of cytotoxic ability) and secretion of IFN-γ (cytokine) in natural killer cells, which target chronic myelogenous leukemia cell line, K562 cells (ATCC® CCL-243™), on day 14 after treatment with IGFBP2 under a hypoxic condition.

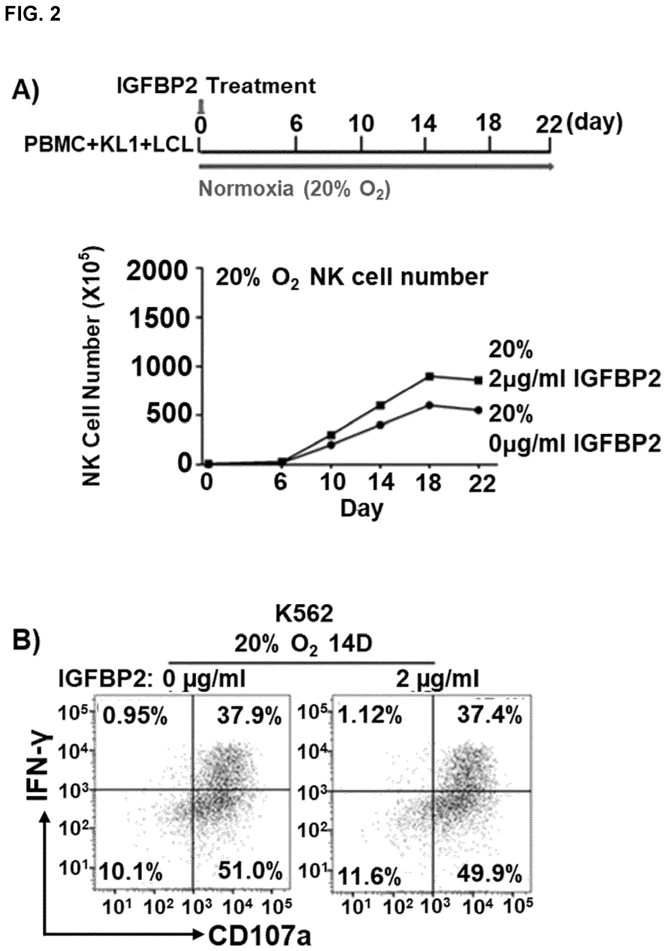

FIG. 2(A) shows a method of culturing natural killer cells in an IGFBP2-containing medium under a normoxic condition, and shows a growth curve of the natural killer cells, and FIG. 2(B) shows the results of analyzing the expression of CD107a (a measure of cytotoxic ability) and secretion of IFN-γ (cytokine) in natural killer cells, which target K562 cells, on day 14 after treatment with IGFBP2 under a normoxic condition.

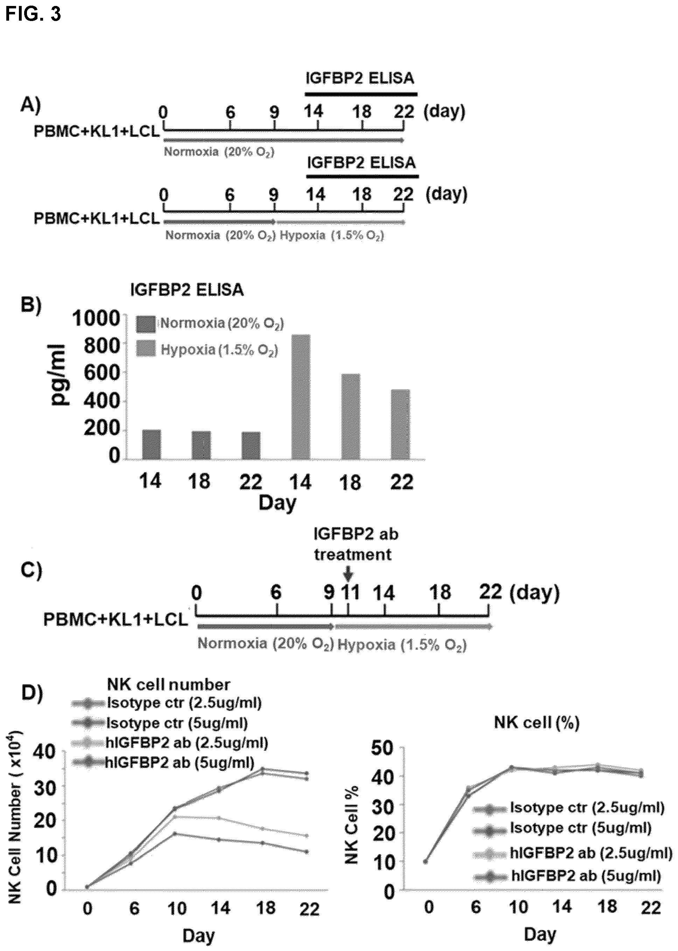

FIG. 3 shows the results of analyzing the effect of IGFBP2, secreted from natural killer cells cultured under a hypoxic condition after culture under a normoxic condition, on proliferation of the natural killer cells. FIG. 3(A) shows a method of measuring IGFBP2 secreted from natural killer cells under a normoxic condition and a hypoxic condition, and FIG. 3(B) shows the results of ELISA (Enzyme-Linked ImmunoSorbent Assay) performed to measure IGFBP2 in a cell culture medium on days 14, 18 and 22 under a normoxic condition and a hypoxic condition according to the method shown in FIG. 3 (A). FIG. 3(C) shows a method of treating a cell culture medium with an antibody for inhibiting IGFBP2, and FIG. 3(D) shows a growth curve indicating that the proliferation of natural killer cells cultured under a hypoxic condition after treatment with an antibody for inhibiting IGFBP2 is inhibited, and shows changes in the distribution of the natural killer cells (Isotype ctr = Isotype Control, hIGFBP2 ab = human IGFBP2 antibody).

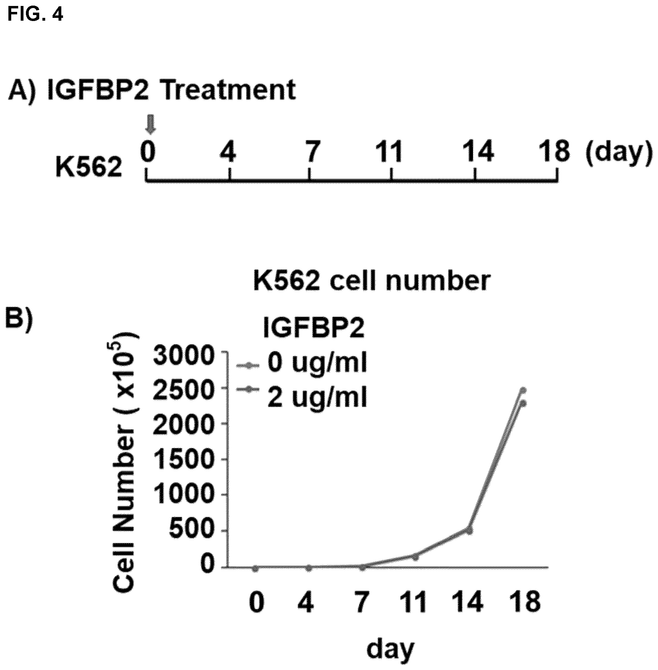

FIG. 4 shows the results of analyzing the effect of IGFBP2 on cancer cell proliferation. FIG. 4(A) shows a method of culturing K562 cancer cells in an IGFBP2-containing medium, and FIG. 4(B) shows a growth curve of the K562 cells cultured according to the method shown in FIG. 4(A).

### Detailed Description and Preferred Embodiments of

### the Invention

Unless otherwise defined, all technical and scientific terms used in the present specification have the same meanings as commonly understood by those skilled in the art to which the present disclosure pertains. In general, the nomenclature used in the present specification is well known and commonly used in the art.

In the present invention, it was confirmed that, when human natural killer cells (NK cells) in the resting state were cultured for expansion under a hypoxic condition (1.5% O₂) and a normoxic condition (20% O₂) from the time point of start of culture (day 0 of culture) up to day 22 in IGFBP2-containing medium, the proliferation of the natural killer cells was enhanced. In addition, it was confirmed that the natural killer cells cultured in IGFBP2-containing medium under the hypoxic condition and the normoxic condition could maintain the cancer cell killing ability thereof regardless of the presence or absence of IGFBP2.

In addition, in connection with the fact that the proliferation of natural killer cells cultured under a hypoxic condition (1.5% O₂) after culture under a normoxic condition (20% O₂) is enhanced (see Korean Patent Publication No. 2017-0124467), it was confirmed that the secretion of IGFBP2 from the natural killer cells cultured under the hypoxic condition significantly increased compared to that from the natural killer cells cultured only under the normoxic condition, and that when the cell culture medium was treated with an antibody for inhibiting IGFBP2, the proliferation of the natural killer cells was inhibited.

Therefore, the present invention is directed to a method for culturing of expansion of natural killer cells comprising culturing natural killer cells in a medium containing IGFBP2.

IGFBP2 (Insulin-like growth factor-binding protein 2) is known as a protein that regulates the function of insulin-like growth factor.

In the present invention, the culturing may be performed for 1 to 22 days.

In the present invention, the culturing may be performed under a hypoxic condition or a normoxic condition.

In the present invention, the hypoxic condition is preferably a condition of 15 to 0.5% O₂, more preferably 5 to 1% O₂.

In the present invention, the normoxic condition is preferably a condition of 18 to 22% O₂, more preferably 19 to 21% O₂, even more preferably 19.9 to 20.9% O₂.

In the present invention, the IGFBP2 may be contained at a concentration of 0.1 to 10 pg/ml, preferably 0.5 µg/ml to 2 µg/ml.

In the present invention, additional culturing under the hypoxic condition or the normoxic condition may be performed for 1 to 30 days.

In an example of the present invention, natural killer cells were transferred to an IGFBP2-containing medium on day 0 of culture and additionally cultured under a hypoxic condition (1.5% O₂) or a normoxic condition (20% O₂) up to 22 days.

In the present invention, the medium may further contain IL-2.

In the present invention, the natural killer cells may be co-cultured. As feeder cells that are co-cultured with the natural killer cells, irradiated Jurkat cells and irradiated EBV-LCL cells may be used.

In the present invention, it was confirmed that, when human peripheral blood mononuclear cells isolated and treated with IGFBP2 at the start of co-culture (day 0 of culture) with Jurkat cells and EBV-LCL cells under a hypoxic condition and a normoxic condition, the number of the natural killer cells was increased compared to that in a conventional culture method.

In addition, based on the fact that the proliferation of natural killer cells, cultured under a normoxic condition (20% O₂) for 9 days and then cultured under a hypoxic condition (1.5% O₂) up to day 22, was enhanced (see the culture method disclosed in Korean Patent Publication No. 10-2017-0124467), the present inventors analyzed the cell culture medium, and as a result, have found that the secretion of IGFBP2 significantly increased compared to that from the natural killer cells cultured only under the normoxic conditions up to day 22. In order to examine whether the secreted IGFBP2 is due to enhanced expansion of proliferating natural killer cells, the cell culture medium was treated with an antibody for inhibiting secreted IGFBP2 and the natural killer cells were cultured. As a result, it was confirmed that the proliferation of the cells decreased.

Additionally, in order to examine the effect of IGFBP2 on cancer cell proliferation, chronic myelogenous leukemia cell line, K562 cells (ATCC® CCL-243™) were treated with IGFBP2 on day 0 of culture and cultured up to day 22, and then the degree of proliferation thereof was measured. As a result, it was confirmed that treatment with IGFBP2 had no effect on the proliferation of the K562 cancer cells.

Hereinafter, the present invention will be described in more detail with reference to examples. It will be obvious to those skilled in the art that these examples serve merely to illustrate the present invention, and the scope of the present invention is not construed as being limited by these examples.

### Example 1: Examination of Increase in Cell Number and Change in Cancer Cell Killing Ability by IGFBP2 Treatment during Culture of Natural Killer Cells under Hypoxic Condition and Normoxic Condition

Human blood was collected, and then centrifuged using Ficoll (Ficoll-Paque™ PLUS, GE Healthcare) at 2500 rpm for 30 minutes, and peripheral blood mononuclear cells were isolated from the buffy coat. Thereafter, the isolated cells were co-cultured with the Jurkat cell line (Korean Cell Line Bank (KCLB): 40152) and EBV-LCL cell line (Korean Cell Line Bank) (each cell line was irradiated with 100 Gy) at a peripheral blood mononuclear cell:Jurkat cell:EBV-LCL cell ratio of 1:0.5:0.5 in hRPMI medium (RPMI1640 medium containing 10% FBS and 1% penicillin/streptomycin) in the presence of 500 U/ml of IL-2. During culture, the medium was replaced with hRPMI medium containing 500 U/ml of IL-2 once every 2 to 3 days.

During culture under the above-described conditions, the cells were cultured under a hypoxic condition (1.5% O₂) from day 0, and the cell culture medium was sampled and secretion of IGFBP2 was analyzed by ELISA (FIG. 1A). When the cells were cultured under the hypoxic condition from day 0 to day 22, there was no significant change in the secretion of IGFBP2 (FIG. 1A).

On day 0 of culture under the above-described conditions, the natural killer cells were treated once with IGFBP2 (Sigma, 0.5 µg/ml to 2 µg/ml) and cultured under a hypoxic condition (1.5% O₂) or a normoxic condition (20% O₂), and the number of the cells was counted (FIGS. 1B and 2A). At this time, the number of the cells was counted using a hematocytometer once every 4 days while the cells were further cultured for about 22 days, and during culture, the medium was replaced with hRPMI medium containing 500 U/ml of IL-2.

As a result, as shown in FIGS. 1B and 2A, it was confirmed that the number of cells in the experimental group treated with IGFBP2 was 1.5 to 4 times larger than that in the control group not treated with IGFBP2.

In addition, in order to examine whether the cancer cell killing ability of the natural killer cells expanded *ex vivo* by the above-described method changes, chronic myelogenous leukemia cell line, K562 cells (ATCC® CCL-243™) were prepared as target cancer cells, and 2.5 µl FITC-labeled anti-CD107a mAb and Golgi stop (BD Pharmingen) were added thereto, followed by culture at 37°C for 5 hours. In the IGFBP2-treated group, IGFBP2 was added once to the cells at a concentration of 2 µg/ml on day 0 of culture, and the cells were cultured in hRPMI medium containing 500 U/ml of IL-2.

After completion of culture, PerCPp-labeled CD3 mAb (eBioscience) and APC-labeled CD56mAb (eBioscience) were added to the cells and allowed to react at 4°C for 20 minutes. For intracellular FACS, the cells were washed with FACS buffer, fixed, permeabilized using a BD Cytoperm/Cytofix kit (BD Pharmingen, San Diego, CA), stained with PE-labeled IFN-g mAb, and then analyzed by flow cytometry.

As a result, as shown in FIGS. 1C and 2B, it could be seen that, in the natural killer cell group treated with the IGFBP2 and the untreated control group, there were no changes in expression of CD107a (a measure of cytotoxic ability) and in secretion of IFN-g (cytokine).

### Example 2: Increase in Cell Number by Extracellularly Secreted IGFBP2 under Hypoxic Condition during Culture of Natural Killer Cells

It was previously found that, when natural killer cells isolated in the same manner as in Example 1 were cultured under a normoxic condition (20% O₂) for 9 days and additionally cultured under a hypoxic condition (1.5% O₂) up to day 22, the proliferation of the natural killer cells was enhanced (see Korean Patent Publication No. 10-2017-0124467). The culture medium of the natural killer cells cultured only under the normoxic condition and the culture medium of the natural killer cells additionally cultured under the hypoxic condition after 9 days of culture under the normoxic condition were collected (each cell culture medium was collected on days 14, 18 and 22 of culture), and IGFBP2 secreted from each type of natural killer cells was measured by ELISA (Enzyme-Linked ImmunoSorbent Assay) (see FIGS. 3A and 3B).

As a result, as shown in FIG. 3B, it was confirmed that secretion of IGFBP2 was 2 to 4 times greater in the natural killer cells additionally cultured under the hypoxic condition than in the natural killer cells cultured only under the normoxic condition.

In addition, the culture medium of the natural killer cells additionally cultured under the hypoxic conditions was treated with an IGFBP2 antibody for inhibiting extracellularly secreted IGFBP2 on day 11 of culture, and then the degree of proliferation of the natural killer cells was examined (FIG. 3C).

As a result, it could be seen that the proliferation of the natural killer cells was inhibited by treatment with the IGFBP2 antibody, and there was no change in the distribution of the natural killer cells (FIG. 3D).

### Example 3: Examination of Effect of IGFBP2 Treatment on Cancer Cell Proliferation

In order to examine the effect of IGFBP2 on cancer cell proliferation, chronic myelogenous leukemia cell line, K562 cells (ATCC® CCL-243™) were treated with IGFBP2 on day 0 of culture and cultured up to day 18 (FIG. 4A). As a result of measuring the degree of proliferation of the cells, it was confirmed that IGFBP2 treatment had no effect on cancer cell proliferation (FIG. 4B). Therefore, it was confirmed that the proliferation of cells by IGFBP2 treatment is applied only to natural killer cells and is not applied to cancer cells.

### Industrial Applicability

When the method for culturing of expansion of natural killer cells according to the present invention is used, the proliferation of human natural killer cells may be significantly enhanced, and thus natural killer cells for use in cancer therapy may be efficiently obtained.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A method for culturing of expansion of natural killer cells comprising culturing natural killer cells in a medium containing IGFBP2.

2. The method of claim 1, wherein the culturing is performed under a hypoxic condition or a normoxic condition.

3. The method of claim 1, wherein the culturing is performed for 1 to 22 days.

4. The method of claim 2, wherein the hypoxic condition is a condition of 15 to 0.5% O₂.

5. The method of claim 4, wherein the hypoxic condition is a condition of 5 to 1% O₂.

6. The method of claim 2, wherein the normoxic condition is a condition of 18 to 22% O₂.

7. The method of claim 1, wherein the IGFBP2 is contained at a concentration of 0.1 to 10 µg/ml.

8. The method of claim 1, wherein the medium further contains IL-2.
